Europäisches Patentamt

European Patent Office

Office européen des brevets

(19)

(11) Publication number: **0 066 917**
**B1**

(12) **EUROPEAN PATENT SPECIFICATION**

(45) Date of publication of patent specification: **14.08.85**

(21) Application number: **82200609.4**

(22) Date of filing: **18.05.82**

(51) Int. Cl.⁴: **A 61 B 6/00, G 01 T 1/164,
B 23 Q 11/00**

(54) **Gamma tomography apparatus comprising a parallelogram suspension system.**

(30) Priority: **29.05.81 NL 8102616**

(43) Date of publication of application:
**15.12.82 Bulletin 82/50**

(45) Publication of the grant of the patent:
**14.08.85 Bulletin 85/33**

(84) Designated Contracting States:
**DE FR GB NL SE**

(56) References cited:
**DE-B-1 516 402
FR-A-2 115 423
FR-A-2 320 723
FR-A-2 336 112
GB-A-2 009 353
US-A-3 892 967
US-A-4 216 381**

(73) Proprietor: **N.V. Philips' Gloeilampenfabrieken
Groenewoudseweg 1
NL-5621 BA Eindhoven (NL)**

(72) Inventor: **Span, Francis Johannes
c/o INT. OCTROOIBUREAU B.V. Prof. Holstlaan 6
NL-5656 AA Eindhoven (NL)**

(74) Representative: **Scheele, Edial François et al
INTERNATIONAAL OCTROOIBUREAU B.V. Prof.
Holstlaan 6
NL-5656 AA Eindhoven (NL)**

Courier Press, Leamington Spa, England.

EP 0 066 917 B1

## Description

The invention relates to a radiation apparatus comprising an element which is to be positioned with respect to an object and which is carried by a balanced suspension system comprising a parallelogram structure to which structure said element to be positioned and a counterweight are suspended from opposite ends so as to balance one another, said parallelogram structure comprising a first arm consisting of two arm sections interconnected by a first interconnecting pivot, the first arm section supporting said element, the second arm section connected to a rod by a pivot.

Such an apparatus which comprises an X-ray tube as radiation source is known from FR—A—2320723. The X-ray is to be positioned with respect to an object to be examined and is carried by a balanced suspension system comprising a counterweight in a parallelogram structure with pivotal supports so as to balance one another. Especially when the element to be positioned is relatively heavy such as a gamma camera in combination with a collimator coupled thereto such as described in US 4,216,381, it should be suspended in a balanced manner by means of a counterweight. In practice, measurements are often impeded by the counterweight, which necessarily also rotates about the patient. Apart from the necessarily heavy construction this drawback is even greater, when an apparatus is to be constructed so that all parts of a patient can be reached thereby.

The invention has for its object to mitigate these drawbacks. To this end, an apparatus of the kind set forth is characterised in that parallel to the first arm two pivotal supports are provided, the first support being connected to said element and to a rotatable supporting ring and the second support being connected to said rotatable ring and to said rod.

In an apparatus in accordance with the invention, the counterweight may be accommodated in a cabinet from which projects only a comparatively long arm which supports the element to be positioned and which due to the double construction of the parallelogram construction, can be easily moved by hand both rotationally and radially with respect to an object arranged on a supporting table. Due to this construction the detector plane of the gamma camera remains oriented in mutually parallel planes during radial movement at any position in the rotary movement.

In a preferred embodiment in accordance with the invention, the moment arm of the counterweight is so short that the entire rotation mechanism and the counterweight can be accommodated in a comparatively narrow closed cabinet. The apparatus thus acquires a more attractive appearance, the more so because an opening is no longer required in the cabinet for the object. An inlet and outlet duct for a fluid to be used during the measurement can then be arranged near the axis.

In a further preferred embodiment, a pivot point for the non-supporting arm is shifted backwards with respect to the element so that more free space for the object is obtained.

The rotation mechanism for the above embodiments may comprise a ring or shaft or a combination of both.

The invention is useful especially for apparatus comprising a gamma camera as a detector but can also be used in, for example, radiation apparatus comprising a heavy radiation source to be balanced, in simulators and in X-rays diagnostic apparatus comprising a rotatably arranged X-ray image intensifier as a detector with a balanced suspension system. In apparatus comprising two measuring heads or one radiation head and one measuring head, the invention can be used separately for both heads in order to avoid the drawback of unbalance which occurs when the adjustment of the heads is not the same. An apparatus comprising one radiation head and one measuring head, which heads have a common counterweight, is known from US—A—3892967.

Some embodiments in accordance with the invention will be described in detail hereinafter. In the drawings:

Figure 1a shows diagrammatically a preferred embodiment of an apparatus according to the invention which comprises a gamma camera carried by a balanced suspension system;

Figure 1b shows the general external appearance of the apparatus;

Figure 2 shows diagrammatically three embodiments of parallelogram suspension systems;

Figure 3 shows diagrammatically three rotation mechanisms for an apparatus in accordance with the invention, comprising a ring, a shaft and a combination thereof, respectively.

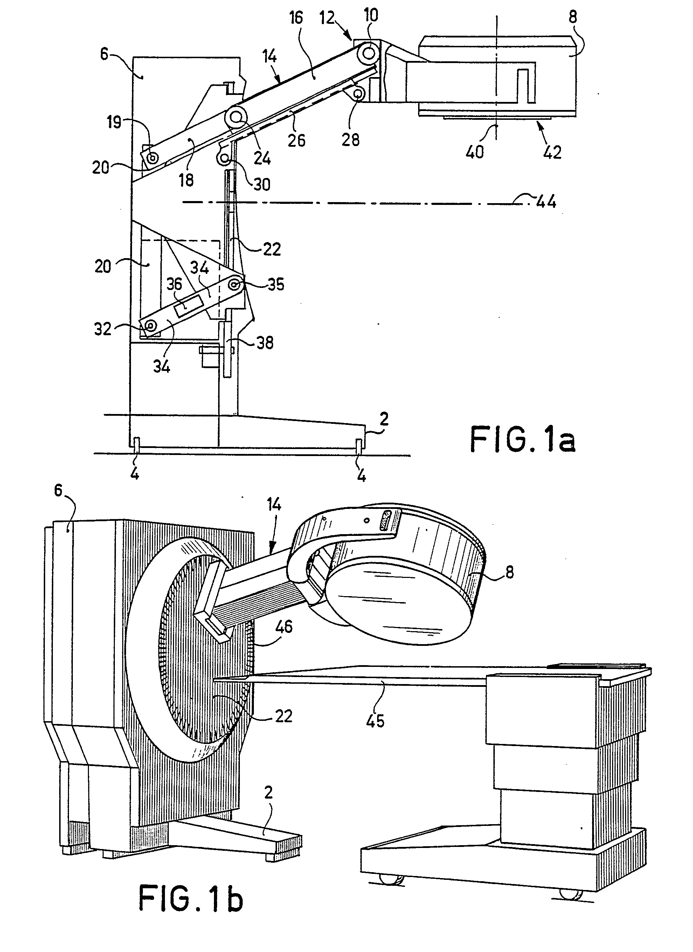

An apparatus as shown in Figure 1 comprises a base 2 which may comprise rollers 4, but which may also alternatively be rigidly connected to the floor. The base supports a housing 6 which is shown in a partly open condition and which accommodates a suspension system for a gamma camera 8. Via a pivot 10, the gamma camera is suspended from a supporting device 12 which comprises a first arm 14 which consists of two sections. A first arm section 16 of the arm 14 extends externally of the housing and supports the gamma camera 8 via the pivot 10. A second section 18 of the arm 14 extends internally of the housing and at one end is connected to a rod 20 by a pivot 19. A pivot 24 which is mounted on a supporting ring 22 interconnects the two arm sections. A second arm 26 is connected at one end to the gamma camera via a pivot 28 and at the opposite end to a pivot 30 which is mounted on the supporting ring 22. The rod 20 is connected via a pivot 32 to a supporting arm 34, which is connected to the supporting ring 22 by a pivot 35. To the supporting arm 34 there is secured a counterweight 36 which is shown as a comparatively small block for the sake of simplicity but which may be composed of, for example, heavy segments. In this embodiment, the supporting ring 22 bears rotatably on, for example, two guide wheels 38 connected to the housing.

Due to the above-described balanced suspension system, the gamma camera can be very easily moved by rotation of the arm 14 about the pivot, even when the arm section 16 is comparatively long so that the camera is spaced far from the housing. In practice, the distance between the near side of the housing and the central axis 40 of the camera will be approximately 1 m, so that a patient can be completely covered by a measurement in two positions. Due to the double construction of the arm section 16, a measuring entry face 42 of the camera is always parallel to a central axis 44, which is the axis of rotation of the supporting ring 24. Normally a patient is positioned so that this axis is the isocentric axis for the patient during measurement. The patient usually rests on a patient table 45 (shown in Figure 1b) which is independently displaceable and adjustable.

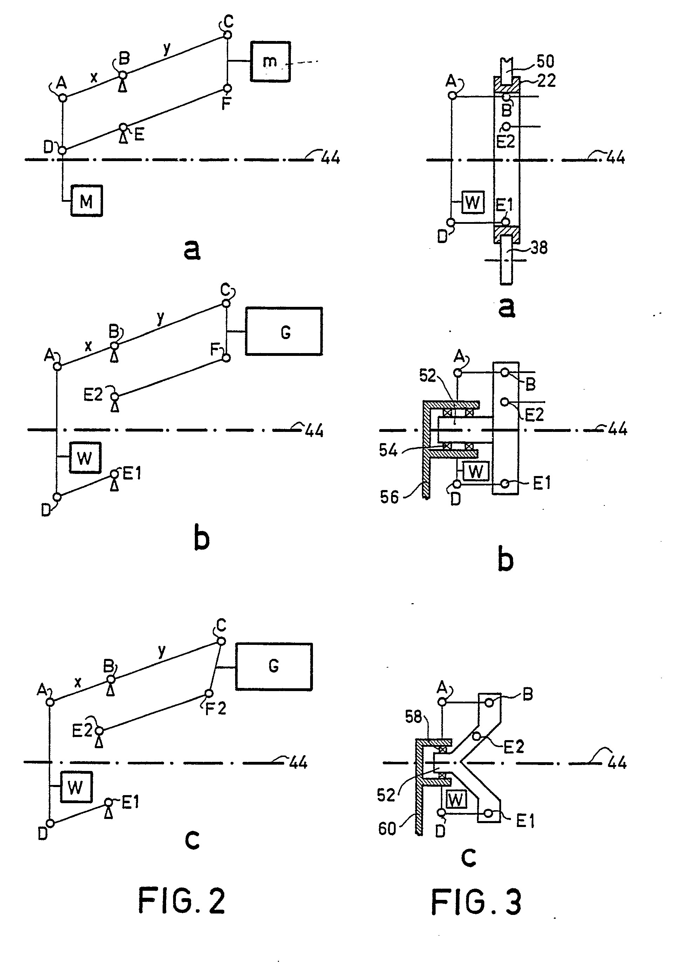

Figure 2 shows diagrammatically, for the sake of clarity, three embodiments of a balanced parallelogram suspension system. The balance between the masses M and m is maintained for as long as $Mx = my$.

During pivoting about points B and E, the points C and F move with one another between mutually parallel planes, so that an element secured thereto maintains a fixed orientation with respect to the axis of rotation 44 during the pivoting movement. Figure 2b shows an arrangement in which a supporting point E has a double construction such that the two functions of the point E, namely supporting and pivoting, are separated. The arm E2-F serves only for the positioning of the gamma camera G, whilst the point E1 provides the support for the counterweight W, so that a much higher degree of freedom is obtained. The structure is thus statically defined and the arm E2-F may have a thin construction so that more free space for the patient is obtained. Even more free space is obtained for the patient by shifting the pivot E2 further to the rear with respect to G, so that this pivot can be situated completely inside the housing.

Figure 3 shows different arrangements for the rotation of the camera. Figure 3a shows the supporting ring 22 of Figure 1 with pivots B, E2 and E1 as shown in Figure 2b supported therein. The supporting ring is supported by guide wheels 38 and rotates in a bearing 50. The supporting ring can be driven by means of a motor (not shown), but due to the efficient balancing and suspension, its displacement requires so little force that manual operation is also possible. Manual operation is usually considered very desirable by the users of apparatus of the kind to which the invention relates. A stepping motor can be used to rotate the gamma camera about the isocentric axis through very accurately adjustable angles, for example, for axial tomography. The supporting ring 22 may be provided with an angular scale division on an outer side. Figure 3b shows an embodiment in which the supporting ring comprises a shaft 52 which is rotatable in a bearing 54 in a supporting bracket 56. Figure 3c shows an embodiment in which the supporting ring 22 has a conical form and again comprises a shaft 52 which is rotatable in a bearing 58 in a supporting bracket 60. In the embodiment shown in Figure 3b, the pivot E2 has been shifted back in accordance with the arrangement shown in Figure 2c. It will be clear from the embodiments described that the invention has a wider field of application than an apparatus of the described kind which comprises a gamma camera as a detection element. The invention is notably suitable, however, for medical diagnostic apparatus in which radiation emitted by the patient is to be measured, because in such apparatus only a single element has to be positioned with respect to the patient. If a source and a detector, for example, an X-ray image intensifier tube has to be moved in a fixed mutual relationship with respect to the patient, such as in X-ray equipment, they can in principle act as a counterweight for one another. If both elements must be independently adjustable with respect to an object to be examined, it is advantageous to accommodate both in a parallelogram suspension system, so that the balance is not disturbed by a difference in adjustment. In addition to diagnostic apparatus, for example, for thermal irradiation the invention can also be used in radiation therapy apparatus. In such apparatus, usually it is merely necessary to position the source with respect to the patient, and a high degree of freedom of movement and a suitably fixed irradiation direction are very important.

**Claims**

1. A radiation apparatus comprising an element (8) which is to be positioned with respect to an object and which is carried by a balanced suspension system comprising a parallelogram structure to which structure said element (8) to be positioned and a counterweight are suspended from opposite ends so as to balance one another, said parallelogram structure comprising a first arm (14) consisting of two arm sections (16, 18) interconnected by a first interconnecting pivot (24), the first arm section (16) supporting said element (8), the second arm section (18) connected to a rod (20) by a pivot (19) characterised in that parallel to the first arm (14) two pivotal supports (26, 34) are provided, the first support (26) being connected to said element (8) and to a rotatable supporting ring (22) and the second support (34) being connected to said rotatable ring (22) and to said rod (20).

2. A radiation apparatus as claimed in Claim 1, characterised in that the supporting ring (22) in which the pivotal supports are mounted is rotatable about an isocentric axis (44) of an object to be examined.

3. A radiation apparatus as claimed in Claim 1, characterised in that the pivot (32) which supports the counterweight (36) is situated on the opposite side of the isocentric axis (44) with respect to the element to be positioned (8).

4. A radiation apparatus as claimed in Claim 1, 2, 3, characterised in that a non-supporting pivot (E2) and the element to be positioned are situated on opposite sides of a plane containing the axes of the supporting pivot (B and E1) the relevant arm length being maintained.

5. A radiation apparatus as claimed in Claim 2, 3 or 4, characterised in that the supporting ring (22) comprises a disk which is supported in a bearing (50) and in which the supporting pivots (B and E1) are accommodated as well as the positioning pivot (E2), which disk is supported by two guide wheels (38).

6. A radiation apparatus as claimed in Claim 5, characterised in that the supporting ring is shaped as a cone and is journalled *via* a shaft (52).

7. A radiation apparatus as claimed in Claim 6, characterised in that the non-supporting pivot (E2) is located in the cone of the supporting ring.

8. A radiation apparatus as claimed in any of the preceding Claims, characterised in that for rotation of the element (8) about an object to be examined there is provided a stepping motor, the supporting ring (22) being provided with an angular scale division.

9. A radiation apparatus as claimed in any of the preceding Claims, characterised in that two elements (8) which are mutually independently adjustable are each carried by a separate parallelogram structure with an individual counterweight for each element.

## Patentansprüche

1. Strahlungsgerät mit einem Element (8), das in bezug auf ein Objekt zu positionieren ist und von einem Gleichgewichts-Aufhängungssystem mit einer Parallelogrammstruktur getragen wird, an deren gegenüberliegende Enden das Element (8) und ein Gegengewicht zum Ausbalancieren aufgehängt sind, wobei die Parallelogrammstruktur einen ersten Arm (14) enthält, der aus zwei Armteilen (16, 18) besteht, die durch einen ersten Verbindungsdrehzapfen (24) miteinander verbunden sind, wobei der erste Armteil (16) das Element (8) trägt und der zweite Armteil (18) über einen Drehzapfen (19) mit einem Stab (20) verbunden ist, dadurch gekennzeichnet, dass parallel zum ersten Arm (14) zwei schwenkbare Träger (26, 34) vorgesehen sind, von denen der erste Träger (26) mit dem Element (8) und mit einem drebaren Tragring (22) und der zweite Träger (34) mit dem drehbaren Ring (22) und mit dem Stab (20) verbunden sind.

2. Strahlungsgerät nach Anspruch 1, dadurch gekennzeichnet, dass der Tragring (22) in dem die schwenkbaren träger montiert sind, um eine isozentrische Achse (44) eines zu untersuchenden Objekts drehbar ist.

3. Strahlungsgerät nach Anspruch 2, dadurch gekennzeichnet, dass sich der Drehzapfen (32), der das Gegengewicht (36) trägt, in bezug auf das zu positionierende Element (8) an der anderen Seite der isozentrischen Achse (44) befindet.

4. Strahlungsgerät nach Anspruch 1, 2 oder 3, dadurch gekennzeichnet, dass sich ein nichttragender Drehzapfen (E2) und das aufzustellende Element an gegenüberliegenden Seiten einer Ebene mit den Achsen des tragenden Zapfens (B und E1) befinden, und dass die relevante Armlänge aufrechterhalten wird.

5. Strahlungsgerät nach Anspruch 2, 3 oder 4, dadurch gekennzeichnet, dass der Tragring (22) eine Schiebe enthält, die von einem Lager (50) unterstützt wird und in der sich die tragenden Drehzapfen (B und E1) sowie der Einstelldrehzapfen (E2) befinden, und dass die Scheibe von zwei Leiträdern (38) getragen wird.

6. Strahlungsgerät nach Anspruch 5, dadurch gekennzeichnet, dass der Tragring kegelförmig ausgeführt und auf einer Achse (52) gelagert ist.

7. Strahlungsgerät nach Anspruch 6, dadurch gekennzeichnet, dass sich der nichttragende Drehzapfen (E2) im kegelförmigen Teil des Tragrings befindet.

8. Strahlungsgerät nach einem der vorangehenden Ansprüche, dadurch gekennzeichnet, dass für die Drehung des Elements (8) um ein zu prüfendes Objekt ein Schrittmotor vorgesehen und der Tragring (22) mit einer Winkelskalenteilung versehen ist.

9. Strahlungsgerät nach einem der vorangehenden Ansprüche, dadurch gekennzeichnet, dass zwei voneinander unabhängige einstellbare Elemente (8) von je einer gesonderten Parallelogrammstruktur mit einem individuellen Gegengewicht für jedes Element getragen werden.

## Revendications

1. Appareil à rayonnement comprenant un élément (8) qui doit être positionné par rapport à un objet et qui est supporté par un système de suspension équilibré comprenant une structure à parallèlogramme à laquelle l'élément (8) à positionner ainsi qu'un contrepoids sont suspendus aux extrémités opposées, de manière à s'équilibrer mutuellement, la structure en forme de parallèlogramme comprenant un premier bras (14) formé de deux sections de bras (16, 18) reliées par un premier pivot de liaison (24), la première section du bras (16) supportant l'élément (8), la seconde section du bras (18) étant reliée à une barre (20) par un pivot (19), caractérisé en ce que parallèlement au premier bras (14) sont prévus deux supports pivotants (26, 34), le premier support (26) étant relié à l'élément (8) et à une couronne de support tournante (22) et le second support (34) étant relié à la couronne tournante (22) et à la barre (20).

2. Appareil à rayonnement suivant la revendication 1, caractérisé en ce que la couronne de support (22) dans laquelle les supports pivotants sont montés peut tourner autour d'un axe isocentrique (44) d'un objet à examiner.

3. Appareil à rayonnement suivant la revendication 2, caractérisé en ce que le pivot (32) qui supporte le contrepoids (36) est situé du côté de l'axe isocentrique (44) opposé à l'élément a positionner (8).

4. Appareil à rayonnement suivant la revendication 1, 2 ou 3, caractérisé en ce qu'un pivot n'assurant pas le support (E2) et l'élément à positionner sont situés de part et d'autre d'un plan passant par les axes du pivot de support (B et E1), la longueur utile du bras étant conservée.

5. Appareil à rayonnement suivant la revendication 2, 3 ou 4, caractérisé en ce que la couronne de support (22) comprend un disque qui est supporté dans un palier (50) et dans lequel les pivots de support (B et E1) sont logés, de même que le pivot de positionnement (E2), ce disque étant supporté par deux galets de guidage (38).

6. Appareil à rayonnement suivant la revendication 5, caractérisé en ce que la couronne de support a la forme d'un cône et est montée à rotation par l'intermédiaire d'un arbre (52).

7. Appareil à rayonnement suivant la revendication 6, caractérisé en ce que le pivot n'assurant pas le support (E2) est placé dans le cône de la couronne de support.

8. Appareil à rayonnement suivant l'une quelconque des revendications précédentes, caractérisé en ce qu'en vue de la rotation de l'élément (8) autour d'un objet à examiner, il est prévu un moteur pas à pas, la couronne de support (22) étant pourvue de graduations formant une échelle d'angles.

9. Appareil à rayonnement suivant l'une quelconque des revendications précédentes, caractérisé en ce que deux éléments (8) qui sont réglables indépendamment l'un de l'autre, sont chacun suppporté par une structure à parallélogramme séparée, un contrepoids individuel étant prévu pour chaque élément.

FIG.1a

FIG.1b

FIG. 2

FIG. 3